# EUROPEAN PATENT APPLICATION

(11) **EP 4 327 663 A2**
(43) Date of publication of application: **28.02.2024**
(21) Application number: 23219317.7
(22) Date of filing: 26.01.2022
(51) Int. Cl.: A23D 7/00, C11C 3/10

(54) **STRUCTURING FATS**

(30) Priority: 26.01.2021 EP 21153504
(62) Divisional of application: 22702680.4
(71) Applicant: Upfield Europe B.V., 1083 HK Amsterdam (NL)
(72) Inventor: DOL, Georg Christian, 1083 HK AMSTERDAM (NL); POTMAN, Ronald Peter, 1083 HK AMSTERDAM (NL); SMIT-KINGMA, Irene Erica, 1083 HK AMSTERDAM (NL)
(74) Representative: EP&C

(57) **Abstract**

The invention relates to a method for providing a structuring fat by the interesterification of a first fat with a high content of saturated long chain fatty acids with a second fat or oil to provide triglyceride compositions having an increased level of H-type fatty acids, especially C18:0 at the 2-position, the triglyceride compositions thus obtained and spreads made therefrom.

## Description

### Field of the invention

The present invention pertains to a structuring fat. The invention also pertains to method for the preparation of the structuring fat. The structuring fat finds application in food compositions such as margarine, spreads and creams.

### Background of the invention

Food compositions comprising fats and oils such as spreads, margarine and the like whether in the form of water in oil (w/o) or oil in water (o/w) emulsions typically contain blends of structuring fats or solid fats and oil (often vegetable oil).

The structuring or solid fat provides the structure and texture to the resulting oil and water emulsion by crystallisation of the triglycerides in the blend. Typically, structuring fats contain more saturated and longer-chain fatty acids.

Vegetable oils typically contain more unsaturated fatty acids and are usually liquid at room temperature. In order to incorporate suitable amounts of fats and oils in consumer products like spread and margarines, blends are made from solid fats (structuring fats) and oils wherein the solid fat (also known in the art as hard stock) provides the structure to incorporate oil into the emulsion.

Hydrogenation is a process of hardening fats and oils by converting unsaturated fatty acids in fats and oils to saturated fats. Hardening of fats is an efficient way of improving the structuring properties of fat and oils. Hydrogenation is perceived by consumers as a non-natural way of adapting fat compositions. Hydrogenation of fats to improve the structure of the resulting fat is less preferred as consumers are increasingly focused on having products that have a more natural origin. Incomplete or partial hydrogenation also results in products having increased levels of trans-fatty acids. Trans-fatty acids are considered less desirable in view of health considerations. There is hence also a need for fat blends that have a low content of trans-fatty acids.

A major source of structuring fats is palm oil-based. The use of palm oil, high in saturated C16 or enriched fractions thereof lies under scrutiny in view of the environmental demand that palm oil exerts. Hence there is a desire to use less palm oil based ingredients and the desire for using other sources of vegetable fats for structuring fats increases. Examples are the use of other plant based fats like shea.

In general, a structuring fat has a high content of saturated fatty acids. Conventional structuring fats or hard stocks have a high saturated content such as in the form of POP or SOS triglycerides. Structuring fats having a high saturated content suffer from what is known in the art as graininess or sandiness, an unpleasant mouthfeel. The effect is attributed to the formation of fat crystals. Furthermore, SSS (tristearic acid glyceride) triglycerides are also seen as cumbersome as in food applications in view of a waxy mouthfeel. Products that express these effects generally experience a lower consumer acceptance level.

The present inventors have set out to develop triglyceride fats that have a relative low level of POP, SOS and/or SSS to avoid the disadvantages thereof or ameliorate the effects thereof, while at the same time having an increased level of saturation desired for structuring the products. Based on these fats, structuring fats or hard stock fats can be developed that are suitable for the development of adequate consumers products such as margarines, spreads etc.

Thus there remains a need for processes to make a structuring fat with even higher amounts of saturated, long chain fatty acids and for structuring fats that have relative high amount of saturated, long chain fatty acids, yet avoid sandiness, graininess or waxiness experiences and have low levels of trans fat, not only in the hard stock, but also in the subsequent applications.

### Summary of the invention

The inventors found that by increasing the amount of stearic acid in the triglyceride, by focussing on increasing the S content and in particular increasing the S content on the 2-position of the glycerol backbone, an improved triglyceride composition could be obtained that can be used as a structuring fat.

The present inventors have found structuring fats (hard stocks) and processes that allow the generation of structuring fats (hard stock) with improved properties and /or applications. The invention encompasses hard stocks that have increased levels of saturated long chain fatty acids (H: S or P) and processes to make such structuring fats having increased levels of saturated long chain fatty acids (H: S or P). The structuring fats of the invention have increased levels of H fatty acids, especially on the 2-position. The structuring fats of the invention have levels of H fatty acids on the 2-position that are significantly higher than comparable hard stocks and correspondingly lower levels of unsaturated fatty acids on the 2-position of the glycerol backbone. The structuring fats have improved (lower) level of trans fatty acids, and can be made from non-palm and non-hydrogenated fats and oils. The structuring fats can be, optionally after further blending with other fats and/ or oils, applied in applications such as spreads and margarines. It was found that fats having a relative higher content of S (and a lower content of P) in the 2-position compared conventional palm based hard stock fats having relative higher levels of P in the 2-position, lead to fat blends that, in spite of a different composition, can be processed under conventional processing conditions yet lead to stable emulsion products that have good organoleptic properties, taste and texture.

Some of the advantages of having structuring fats available that have a higher content of saturated long chain (H) fatty acids, preferably stearic acid (S) containing structuring fats are that lower amounts of structuring fats can be needed and more liquid oil can be used in the preparation of applications for spreads and margarines and the like. This further improves the naturalness of the resulting product, improves nutritional value and also reduces cost, which is a relevant factor in this field.

One aspect of the invention is an interesterification process for making an triacylglyceride composition comprising an interesterification step of a first fat comprising more than 70 wt.% H-type fatty acids, drawn on the total amount of first fat, with a second fat to provide a triacylglyceride composition that can be used as a structuring fat.

Another aspect resides in the triglyceride composition, optionally obtainable by the method.

A further aspect resides in a hard stock fat or structuring fat based on the triglyceride composition of the invention.

Yet another aspect is in the use of a triacylglyceride composition as a hard stock or structuring fat and its use in the preparation of a water-continuous or fat-continuous edible emulsion. A further aspect of the invention is found in a margarine or a spread comprising a triglyceride composition.

### Detailed description

The term "oil" or "liquid oil" is typically used for triglyceride compositions that that are liquid at room temperature. The term "liquid oil" is used for triglycerides that are liquid at room temperature, preferably also liquid at temperature below room temperature such as below 15, 10 or 5° C. Preferably the solid fat content of the liquid oil is 0 at 20° C, more preferably it is 0 at 15° C.

The term "fat", is typically used for triglyceride compositions that that are solid at room temperature. The use of the term "oil" or "fat" is hence interchangeable depending on the circumstances that are clear and known in the art.

A fat is typically used for structuring a fat composition, i.e. to provide a structure and texture in admixture with an oil or other fat. It can also be indicated as a structuring fat or hard stock fat The fat may comprise two or more different hard fats (a blend), but is preferably a single fat. The fat may be an interesterified mixture of one or more fats.

A "margarine fat" is a fat blend which is suitable for use as a fat in spreads, both fat-continuous and water-continuous, such a margarine fat usually includes a fat and a liquid oil. Solid fats from which lower melting constituents have been removed are typically indicated as "stearin fractions". A stearin fraction for the purpose of this description is defined as a triglyceride mixture or fat blend from which at least 10% of the lower melting constituents have been removed by some kind of fractionation, e.g. dry fractionation, multi-stage countercurrent dry fractionation or solvent fractionation. The lower melting constituents are indicated as "olein fraction".

The fat fraction can also be characterized by a triacylglyceride or TAG profile. In the TAG profile and throughout this application, the following abbreviations are used:

| | |
|---|---|
| M | Medium chain fatty acid (C12-C14) |
| H | Saturated long chain fatty acid >= C16 |
| P | Palmitic acid C16:0 |
| S | Stearic acid, C18:0 |
| U | Unsaturated fatty acid |
| O | Oleic acid C18:1 |
| L | Linoleic acid/linolenic acid C18:2,C18:3 |
| St | Saturated fatty acid |
| StStSt | Trisaturated glyceride |
| StU2 | Monosaturated triglyceride |
| StU St | Symmetrical saturated triglyceride |
| St2U | Disaturated triglyceride |
| HUH | Symmetrical long chain triglyceride |
| H2U | Disaturated long chain triglyceride |
| HU2 | Monosaturated long chain triglyceride |
| U3 | Unsaturated triglyceride |
| H3 | Long chain saturated fatty acid triglyceride |
| H2M | Mixed (2 long, 1 medium) chain fatty acid triglyceride |
| M3 | Medium chain fatty acid triglyceride |
| Sh | Short chain fatty acid (C4-C10) |
| X | Any fatty acid (H, U or Sh) |

In this specification all parts, proportions and percentages are by weight; the amount of fatty acids in an oil or fat is based on the total amount of fatty acids in the oil or fat and the amount of fat in the fat composition is based on the total weight of the fat composition, unless otherwise stated.

The solid fat content (SFC) in this description and claims is expressed as N-value, essentially as defined in Fette, Seifen Anstrichmittel 80 180-186 (1978). The stabilisation profile applied is heating to a temperature of 80 °C., keeping the oil for at least 10 minutes at 60 °C. or higher, keeping the oil for 16 hours at 0 °C. and then 30 minutes at the measuring temperature, except where indicated otherwise.

Non-hydrogenated means that the fat or oil has not undergone hydrogenation treatment. This entails the fats as well as blends and interesterified mixtures of the fats. Non-hydrogenated fats have essentially no trans-fatty acids. Preferably the fat of the invention has less than 5 wt.%, preferably less than 2 wt.%, of trans fatty acids, more preferably less than 1 wt.%, 0.5, 0.1 wt.% or even 0 wt.% (non-detectable using analysis methods common in the art).

Conventionally partially hardened fat typically has as one of its disadvantages a relative high level of trans fat. Conventionally partially hardened fats are made by partial hydrogenation of an oil, typically a highly unsaturated oil. A highly unsaturated oil, such as sunflower oil, contains a high amount of unsaturated fatty acids (typically more than 90%), also at the 2-position. Partial hardening through hydrogenation is known to lead to the formation of trans-fatty acids, also at the 2-position. The fat of the invention is obtained by transesterification or glycerolysis with saturated free fatty acids. This avoids trans-fat formation and leads to a fat that has an inherently lower trans-fat level, also on the 2- position. At the same time, the fat of the invention can still contain a certain level of unsaturated fatty acids.

In the known highly saturated fats, the fat has appeared to crystallize as coarse grains, which are unacceptable for a spread which ought to possess a smooth appearance and mouthfeel. Such effect is known in the art as sandiness or graininess.

In the case of sandiness the particles have higher melting points, they do not melt so readily when rubbing them between the fingers. The well-known graininess consists of particles which also melt at relatively low temperature but the particle sizes are much smaller. It is known in the art that such effects are caused by fats that are high in POP and SOS.

By increasing the level of saturation at the 2-position using the method of the invention, the formation of POP and SOS is reduced and the method and resulting fat of the invention thus avoids the sandiness or graininess. This is determinable for instance by mouthfeel or by microscopy. At the same time due to the presence of a certain amount of unsaturation, the waxiness mouthfeel known from other fats that are highly saturated such as SSS or fully saturated sunflower oil (SF69) can be avoided.

Interesterification and transesterification are a methods for adapting the fatty acid composition of a fat composition.

Interesterification as used in the present disclosure and distinguished in the present disclosure from transesterification refers to the exchange of fatty acids between triglycerides in a triglyceride mixture. In interesterification, the total fatty acid composition of the triglyceride mixture remains substantially the same, yet the distribution of the fatty acids over the glycerol backbone may be different. Interesterification typically results in a redistribution of the fatty acids over the glycerol backbone.

Transesterification, as used in the present disclosure and distinguished in the present disclosure from interesterification, refers to the exchange of fatty acids between fatty acids (or fatty acid esters) and triglycerides. In transesterification, the total fatty acid composition of the triglyceride mixture changes. Transesterification results in a different fatty acid composition of the triglyceride mixture.

Thus in a first aspect, the invention relates to a method for making an triacylglyceride composition comprising an interesterification step of a first fat comprising more than 70 wt.% H-type fatty acids, drawn on the total amount of first fat, with a second fat to provide a triacylglyceride composition. In the method of the invention a first fat is used that has a high content (more than 70 wt.%) of long chain saturated fatty acids of the H- type (C16:0, C18:0). Preferably the first fat has a content of more than 70 wt.% of the H3-type, preferably of the SSS type. The first fat is preferably characterised by having a high level of at least 70 wt. % of H-type fatty acids at the 2-position, preferably of C18:0. The first fat may be non-hydrogenated and/or palm oil free. The second fat is an edible oil or fat, preferably selected from the group consisting of coconut oil, sunflower oil, rapeseed oil, shea and fractions thereof, linseed, palm oil and fractions thereof and palm kernel and fractions thereof, and mixtures thereof.

The first fat and the second fat can be blended in ranges from 1 wt.% of the first fat with 99 wt.% of the second fat to 99 wt.% of the first fat to 1 wt.% of the second fat depending on the desired triglyceride composition to be used as a structuring fat. The ratios and one or more components of the second fat may be selected in relation to the first fat such that fatty acid profiles (FA distributions) are achieved that contain desired levels of short chain, medium chain and long chain length fatty acids. There is a preference for an amount of the first fat drawn on the total fat from 10 to 65 wt.%. Using the first fat as defined herein (a high amount of H-type fatty acids especially at the 2-position), a lesser amount of the first fat is needed to come to desirable structuring fats while profitable characteristics of the resulting structuring fat can still be achieved.

The first fat and second fat can be blended and subjected to interesterification conditions. The interesterification may be enzymatic or catalytic, respectively and can be carried out under conventional conditions. Prior to and/or after completion of the interesterification, the blend or the resulting triglyceride composition can be subjected to neutralisation and/or deodorisation steps.

In second aspect, the triglyceride composition, optionally obtained by the method of the invention, is particularly characterised in having a relative high content of saturated long-chain fatty acids (H-type, especially C18:0) and in particular at the 2-position compared to conventional structuring fats. The triglyceride composition of the present invention is particularly characterised by having a relative low P-content and/or relative high S content at the 2-position. The triglyceride composition of the present invention can also be characterised by having a relative low U-content and/or relative high H content at the 2-position.

This can be expressed in various ratio's as shown in the embodiments and examples.

In preferred embodiments, the triglyceride composition is characterised as having a 2-C18:0 content of between 15 and 40 wt.% drawn on the total fatty acid content on the 2-position, optionally combined with a 2-C16:0 content of between 5 and 15 wt.% drawn on the total fatty acid content on the 2-position. The triglyceride composition can also, or in combination with the 2-position C18:0 and/or C16:0 profile, further characterised as having a total C18:0 content of between 20 and 45 wt.%, drawn on the total fatty acid content, optionally in combination with a C16:0 content of between 5 and 10 wt.%, drawn on the total fatty acid content. Other embodiments and their combinations are listed in the embodiments section below.

The triglyceride composition of the present invention provides for at least similar structuring properties but decreased sandiness/graininess due to the lower presence of at least POP triglycerides.

The triglyceride composition or structuring fat of the invention is capable of being blended with other fats and oils to create water-continuous or fat-continuous edible emulsions that can be used as such as margarines or spreads. The triglyceride composition or structuring fat of the invention is also capable of being used in (ice) creams, dairy alternatives, such as non-dairy creams and food applications in general.

### Examples:

### Characterization of the starting component and the reaction product

The products resulting from the interesterification were analysed using the following analytical methods:

### 1. FA analysis

For any starting fat or product, the overall fatty acid analysis and the triglyceride composition is determined using conventional procedures in the art such as FAME analysis, GLC/Carbon number method and HPLC silver phase method such as described for example in EP78568, EP652289, JOACS (19914), 68(5), 289-293 and Hammond E.W.J., Chromatography, 203, 397, 1981.

### 2. Solid Fat Content (SFC) measurements

The solid fat content (SFC) in this description and claims is expressed as N-value, as defined in Fette, Seifen Anstrichmittel 80 180-186 (1978). The stabilization profile applied is heating to a temperature of 80 degrees Celsius, keeping the oil for at least 10 minutes at 60 degrees Celsius or higher, keeping the oil for 1 hour at 0 degrees Celsius and then 30 minutes at the measuring temperature (tempered). An alternative method is described in IUPAC 2.150 method, serial, non-tempered.

### 3. 2-Position analysis

The method is based on the Joint JOCS/AOCS Official Method Ch 3a-19 (2019).

This method provides a procedure for the determination of the composition of fatty acids which are esterified at the sn-2 position (β (beta) or internal position) of the triacylglycerol molecules in animal and vegetable fats and oils. The method is comprised of the 1 (3)-position selective transesterification of the triacylglycerols with ethanol by *Candida antarctica* lipase to yield 2-monoacylglycerols, followed by the separation of the 2-monoacylglycerols by silica-gel chromatography, and determination of their fatty acid composition by gas chromatography.

### Feed stock Glycerolysis

A feed stock was obtained by reacting an excess of stearic acid with glycerol under increased pressure and temperature in a conventional way (24hrs, 150-220 degrees Celsius, reduced pressure). After completion of the reaction, the excess stearic acid was removed by stripping to yield a fat with a C18:0 content of 96.4% and a CN54 of 90%. Data of the feedstock used are presented in Table 1.

### Hard stock

The feedstock of Table 1 is interesterified with other oils and fats (sunflower oil, coconut oil) to produce a hard stock (Examples 1-8) that can be applied e.g. in margarine fat, margarine and spreads production. Conventional palm-based hard stock is presented as Comparative Examples 1 and 2. Interesterification is executed by chemical interesterification (CIE) with a catalyst (NaOCH₃) or by enzymatic interesterification (EIE) with lipase, for example Lipozyme TLIM (NOVOZYME). The ratio of the feedstock and the other oils and fats is selected to optimize the properties of the desired hard stock as an ingredient in a final application e.g. margarine fat, margarine and spread.

### Chemical Interesterification Procedure (CIE)

The feed stock is blended with other oils and fats in an appropriate ratio as specified to come to the desired hard stock. A stoichiometric amount of NaOH (50% w/w solution in water) is added to ensure FFA of the blend is <0.05% before catalyst dosing. Once the oil is free of FFA, the oil is dried under vacuum to eliminate any residual water (<100 ppm as measured by Karl Fisher titration). The catalyst is added (0.1% w/w sodium methoxide) and the blend brought under vacuum (25 mbar). The reaction starts when the color of the blend darkens (red/brownish).Once the reaction is started, the reaction is continued for 30 minutes at 90°C and 25 mbar. After completion of the reaction, the catalyst is inactivated by breaking the vacuum in the reactor and adding citric acid. The acid is dosed to ensure 20% molar excess vis-a-vis added catalyst. Citric acid is dosed as a 20% solution (w/w in water). The mixture is allowed to react for 15 minutes at 90°C at atmospheric pressure. The CIE hard stock is post-treated: bleached and deodorized. The resulting hard stock is analysed for TAG profile, N-line, 2-position FA analysis.

### Enzymatic Interesterification Procedure (EIE)

The feed stock is blended with other oils and fats in an appropriate ratio as specified to come to the desired hard stock. To the blend 8% of Lipozyme TLIM (Novozyme) is added. The mixture is allowed to react at 70°C, atmospheric pressure and under agitation to keep the enzyme in suspension for 24 hours. The enzyme is filtered off.

The EIE hard stock is post-treated: bleached and deodorized. The resulting hard stock is analysed for TAG profile, N-line, 2-position FA analysis.

### Bleaching

The oil is heated to 85°C. A stoichiometric amount of citric acid (30% solution) is dosed to acidulate soaps and the mixture reacted for 15 minutes at atmospheric pressure. The oil is heated to 100°C under vacuum (50 mbar). Bleaching earth (1% - Tonsil 210FF or equivalent activated clay) is added. The slurry is agitated for 30 minutes, at 100°C and 50 mbar. The bleaching earth is filtered off.

### Deodorization

The bleached oil is deodorized until FFA< 0.05% (indicative parameters: 240°C - 3 mbar-0.5-1% stripping steam). Cool down to 80°C followed by purging with nitrogen and storage of the hard stock in hermetically closed recipients.

### Emulsification

Fats and spreads are prepared by blending fats, oils and/or aqueous phases using conventional procedures such as described in handbooks like "Fats and Oils Handbook", 1998, Michael Bockisch.

### Example 9 Margarine fat

A margarine fat is prepared by blending 70% sunflower oil with 30% of the hard stock fat of Example 1.

### Example 10 Margarine fat

A margarine fat is prepared by blending 20% sunflower oil with 80% of the hard stock fat of Example 7.

### Example 11 Margarine fat

A margarine fat is prepared by blending 55% rapeseed oil with 45% of the hard stock fat of Example 7.

### Example 12 Spread

A spreadable margarine in a tub is prepared with the hard stock of example 1 with the following ingredients:

| | % | % | |
|---|---|---|---|
| Margarine fat Example 9 | | 59.49 | |
| Sunflower oil | 70 | | |
| Hard stock example 1 | 30 | | |
| monoglyceride | | 0.3 | |
| Lecithine | | 0.2 | |
| Minor components (fat) | | 0.01 | |
| Total | | | 60 |
| Water | | 39.9 | |
| Minor components (water) | | 0.1 | |
| Total | | | 40 |

### Example 13 Spread

A light margarine is prepared with the hard stock of example 1 7 with the following ingredients:

| | % | % | |
|---|---|---|---|
| Margarine fat Example 9 | | 39.14 | |
| Sunflower oil | 70 | | |
| Hard stock Example 7 | 1230 | | |
| monoglyceride | | 0.35 | |
| Lecithine | | 0.5 | |
| Minor components (fat) | | 0.01 | |
| Total | | | 40 |
| Water | | 59.9 | |
| Minor components (water) | | 0.1 | |
| Total | | | 60 |

### Example 14 Spread

A block margarine in a wrapper is prepared with the hard stock of example 7 with the following ingredients:

| | % | % | |
|---|---|---|---|
| Margarine fat Example 10 | | 79.59 | |
| Sunflower oil | 20 | | |
| Hard stock example 7 | 80 | | |
| Lecithine | | 0.4 | |
| Minor components (fat) | | 0.01 | |
| Total | | | 80 |
| Water | | 19 | |
| Minor components (water) | | 1 | |
| Total | | | 20 |

### Example 15 Spread

A spreadable margarine in a tub is prepared with the hard stock of example 7 with the following ingredients:

| | % | % | |
|---|---|---|---|
| Margarine fat example 11 | | 79.39 | |
| Rapeseed oil | 55 | | |
| Hard stock example 7 | 45 | | |
| Lecithine | | 0.6 | |
| Minor components (fat) | | 0.01 | |
| Total | | | 80 |
| Water | | 18.5 | |
| Minor components (water) | | 1.5 | |
| Total | | | 20 |

Stability tests are performed for 2-3 weeks cycling at 12 hrs/20 degrees Celsius, 12 hours/5 degrees Celsius. Every week droplet size, spreadability and visual check on emulsion break down was performed. Mouthfeel is positively tested (no waxiness) as is taste and texture. The products expressed a good stability, breakdown of emulsion, and no occurrence of graininess or sandiness is observed.

**Table 1**

| | **CN** | **SF** | **FS** | **Ex 1** | **Ex 2** | **Ex 3** | **Ex 4** | **Ex 5** | **Ex 6** | **Ex 7** | **Ex 8** | **Ex 9** | **C1** | **C 2** |
|---|---|---|---|---|---|---|---|---|---|---|---|---|---|---|
| **Interesterification** | | | | **CIE** | **CIE** | **ElE** | **CIE** | **CIE** | **CIE** | **CIE** | **EIE** | **CIE** | | |
| **Blend** | | | | 35FS/ 20SF/ 45CN | 35FS/ 20SF/ 45CN | 35FS/ 20SF/ 45CN | 25FS/ 30SF/ 45CN | 20FS/ 25SF/ 55CN | 25FS/ 30SF/ 45CN | 20FS/ 25SF/ 55CN | 25FS/ 30SF/ 45CN | 50CN/ 50FS | 65dfPOs/ 35PK | 50PO/ 10dfPOs/ 40PK |
| **FAC %** | | | | | | | | | | | | | | |
| C8:0 | 7,3 | | | 3,1 | 3,4 | 3,3 | 3,3 | 4 | 3,3 | 3,9 | 3 | 3,6 | 1,3 | 1,5 |
| C10:0 | 5,9 | | | 2,5 | 2,7 | 2,7 | 2,7 | 3,2 | 2,6 | 3,2 | 2,4 | 2,9 | 1,2 | 1,4 |
| C12:0 | 45,1 | <0.1 | | 19,9 | 20,7 | 20,5 | 21,3 | 24,4 | 20,2 | 25 | 18,4 | 23,7 | 16,2 | 18,8 |
| C14:0 | 18,4 | 0,1 | | 8,2 | 8,4 | 8,3 | 8,7 | 10 | 8,2 | 10,3 | 7,5 | 9,3 | 6,4 | 7,2 |
| C16:0 | 9,7 | 6,4 | 0,9 | 8,4 | 5,6 | 5,7 | 7,3 | 8,1 | 7,9 | 8,8 | 6,2 | 5,8 | 42,1 | 31 |
| C18:0 | 3 | 4,1 | 96,4 | 32,3 | 36 | 35,8 | 25,4 | 20,7 | 24,5 | 20,5 | 26,5 | 47,3 | 4 | 3,7 |
| C18:1 | 7,3 | 33,2 | 0,4 | 11,2 | 8,4 | 8,6 | 13,5 | 12,6 | 14 | 12,9 | 11,9 | 3,9 | 22,7 | 28,4 |
| C18:2 | 2 | 53,9 | 0,1 | 12,5 | 13,4 | 13,5 | 16 | 15,1 | 17,1 | 13,5 | 22,5 | 1,0 | 4,8 | 6,7 |
| C20:0 | <0.1 | 0,3 | 1 | 0,4 | 0,4 | 0,4 | 0,4 | 0,6 | 0,7 | 0,5 | 0,3 | 1,0 | 0,3 | 0,3 |
| C22:0 | | 0,8 | 0,5 | 0,2 | 0,2 | 0,2 | 0,3 | 0,2 | 0,3 | 0,4 | 0,3 | 0,6 | | |
| SAFA | 90,5 | 12,1 | 99,1 | 75,7 | 77,8 | 77,6 | 69,9 | 71,7 | 68,2 | 73,3 | 65,1 | | 72 | 64,3 |
| MUFA | 7,4 | 33,6 | 0,4 | 11,4 | 8,5 | 8,7 | 13,7 | 12,8 | 14,2 | 13 | 12,1 | | 22,9 | 28,6 |
| PUFA | 2 | 54,1 | 0,2 | 12,7 | 13,5 | 13,6 | 16,2 | 15,2 | 17,3 | 13,6 | 22,6 | | 4,9 | 6,9 |
| Total trans content | <0.5 | <0.5 | <0.5 | <0.5 | <0.5 | <0.5 | <0.5 | <0.5 | <0.5 | <0.5 | <0.5 | | <0.5 | <0.5 |

| **N lin e%** | | | | | | | | | | | | | | |
|---|---|---|---|---|---|---|---|---|---|---|---|---|---|---|
| N5 | | | 96.6 | 78.1 | 79.7 | 79,8 | 67.3 | 67,8 | 68,2 | 73,1 | 61,9 | | 81,3 | 67,5 |
| N 10 | | | 96.4 | 73.1 | 75.1 | 75,4 | 61.1 | 59,8 | 61,7 | 66,3 | 54,8 | | 78,1 | 60,2 |
| N 15 | | | 96.1 | 61.2 | 63.4 | 63,5 | 46.4 | 44,8 | 46,5 | 49,8 | 38,9 | | 66,8 | 44,5 |
| N 20 | | | 95.7 | 48.5 | 50.6 | 49,3 | 33.3 | 31,1 | 32,5 | 35,3 | 26,1 | | 53,7 | 30,9 |
| N25 | | | 95.6 | 35 | 36.4 | 35,4 | 20.6 | 16,2 | 19,3 | 19,2 | 15,3 | | 40,4 | 18,4 |
| N30 | | | 95.7 | 22.2 | 22.7 | 21,8 | 10.1 | 5,9 | 9,1 | 7,2 | 7,1 | | 26,5 | 8,9 |
| N35 | | | 95.4 | 11.6 | 10.8 | 10,4 | 4.1 | 1,4 | 3,4 | 1,1 | 2,5 | | 15,1 | 3,0 |

| **Silverphase %** | | | | | | | | | | | | | | |
|---|---|---|---|---|---|---|---|---|---|---|---|---|---|---|
| StStSt | | | 98,4 | 53,4 | | | 50,6 | 47,4 | 41,4 | 49 | | | 41,6 | 30,5 |
| SOS | | | 0,1 | 7,2 | | | 8,5 | 2,8 | 2,5 | 5,5 | | | 8,8 | 8,4 |
| StStO | | | | 8,6 | | | 11,5 | 15,6 | 16,7 | 15,4 | | | 28,2 | 31 |
| StLSt | | | | 3 | | | 2,8 | 3,4 | 3,5 | 2,5 | | | 3 | 3,6 |
| StStL | | | | 10 | | | 10,3 | 9,9 | 10,9 | 9,1 | | | 2 | 1,8 |
| StOO | | | | 5,1 | | | 6,1 | 6,6 | 7 | 6,6 | | | 7,1 | 9,6 |
| OStO | | | | 1,3 | | | 0,7 | 2,9 | 1 | 2,9 | | | 3,9 | 6,1 |
| StLO/StOL/OStL | | | | 4,3 | | | 3,5 | 5,6 | 6 | 3,5 | | | 3,1 | 4,9 |
| OOO | | | 0,3 | 2,4 | | | 1,4 | 0,2 | 2,3 | 1,3 | | | 1,1 | 2,2 |
| REST | | | 0,7 | 3,8 | | | 3,9 | 4,7 | 7,3 | 3,6 | | | 1 | 1,6 |
| Unknown | | | 0,5 | 0,9 | | | 0,6 | 0,8 | 1,2 | 0,6 | | | 0,2 | 0,4 |
| H | 12,7 | 10,5 | 97,3 | 40,7 | 41,6 | 41,5 | 32,7 | 28,8 | 32,4 | 29,3 | 32,7 | 53,1 | 46,1 | 34,7 |
| M | 63,5 | 0,1 | 0 | 28,1 | 29,1 | 28,8 | 30 | 34,4 | 28,4 | 35,3 | 25,9 | | 22,6 | 26 |
| U | 9,3 | 87,1 | 0,5 | 23,7 | 21,8 | 22,1 | 29,5 | 27,7 | 31,1 | 26,4 | 34,4 | 4,9 | 27,5 | 35,1 |
| StU U | | | 0 | 5,1 | | | 6,1 | 6,6 | 7 | 6,6 | | | 7,1 | 9,6 |
| StStU | | | 0 | 18,6 | | | 21,8 | 25,5 | 27,6 | 24,5 | | | 30,2 | 32,8 |
| StStSt | | | 98,4 | 53,4 | | | 50,6 | 47,4 | 41,4 | 49 | | | 41,6 | 30,5 |
| StUU+StStU+StStSt | | | 98,4 | 77,1 | | | 78,5 | 79,5 | 76 | 80,1 | | | 78,9 | 72,9 |
| StStU+StStSt | | | 98,4 | 72 | | | 72,4 | 72,9 | 69 | 73,5 | | | 71,8 | 63,3 |
| StStU+StStSt/ (StUU+StStU+StStSt) | | | 1 | 0,93 | | | 0,92 | 0,92 | 0,91 | 0,92 | | | 0,91 | 0,87 |
| St3 | | | 98,4 | 53,4 | | | 50,6 | 47,4 | 41,4 | 49 | | | 41,6 | 30,5 |
| St2X | | | 98,5 | 82,2 | | | 83,7 | 79,1 | 75 | 81,5 | | | 83,6 | 75,3 |
| St2 U | | | 0,1 | 28,8 | | | 33,1 | 31,7 | 33,6 | 32,5 | | | 42 | 44,8 |
| StU2 | | | 0 | 10,7 | | | 10,3 | 15,1 | 14 | 13 | | | 14,1 | 20,6 |

| **2-position %** | | | | | | | | | | | | | | |
|---|---|---|---|---|---|---|---|---|---|---|---|---|---|---|
| C8:0 | | | | 3 | | | 3,1 | 5 | 3,9 | 1,2 | | | 0,8 | 0,7 |
| C10:0 | | | | 2,6 | | | 2,8 | 4 | 3,3 | 2,5 | | | 1 | 1,1 |
| C12:0 | | | 0,2 | 21,5 | | | 22,3 | 28,8 | 23,7 | 25,2 | | | 17,6 | 21,4 |
| C14:0 | | | 0,1 | 8,5 | | | 8,9 | 10,4 | 8,7 | 11,1 | | | 6,4 | 7,1 |
| C16:0 | | | 1,3 | 9,1 | | | 8,8 | 7,8 | 7,8 | 9,9 | | | 36,6 | 21 |
| C18:0 | | | 95,8 | 34,6 | | | 28 | 19 | 23,5 | 23,2 | | | 2,9 | 2,1 |
| C18:1 | | | 0,6 | 9,3 | | | 11,3 | 11,2 | 12,7 | 13 | | | 28,6 | 37,4 |
| C18:2 | | | 0,4 | 9,5 | | | 12,8 | 11,8 | 14,2 | 12,8 | | | 5,9 | 9 |
| C20:0 | | | 0,9 | 0,5 | | | 0,4 | 0,5 | 0,6 | 0,6 | | | | |
| C22:0 | | | 0,4 | 0,2 | | | 0,2 | 0,2 | 0,2 | 0,4 | | | | |
| 2-H (2-C16:0+2-C18:0) | | | 97,1 | 43,7 | | | 36,8 | 26,8 | 31,3 | 33,1 | | | 39,5 | 23,1 |
| 2-C16:0/2-H | | | 0,0 | 0,2 | | | 0,2 | 0,3 | 0,2 | 0,3 | | | 0,9 | 0,9 |
| 2-C18:0/2-H | | | 1,0 | 0,8 | | | 0,8 | 0,7 | 0,8 | 0,7 | | | 0,1 | 0,1 |
| 2-U (C18:1+C18:2) | | | 1,0 | 18,8 | | | 24,1 | 23,0 | 26,9 | 25,8 | | | 34,5 | 46,4 |
| 2-U/2-H | | | 0,0 | 0,4 | | | 0,7 | 0,9 | 0,9 | 0,8 | | | 0,9 | 2,0 |
| 2-C18:0/2-U | | | 95,8 | 1,8 | | | 1,2 | 0,8 | 0,9 | 0,9 | | | 0,1 | 0,0 |
| 2-C16:0/2-U | | | 1,3 | 0,5 | | | 0,4 | 0,3 | 0,3 | 0,4 | | | 1,1 | 0,5 |
| 2-C18:0/2-C16:0 | | | 73,7 | 3,8 | | | 3,2 | 2,4 | 3,0 | 2,3 | | | 0,1 | 0,1 |
| 2-C16:0/total C16:0 | | | 1,4 | 1,1 | | | 1,2 | 1,0 | 1,0 | 1,1 | | | 0,9 | 0,7 |
| 2-C18:0/total C18:0 | | | 1,0 | 1,1 | | | 1,1 | 0,9 | 1,0 | 1,1 | | | 0,7 | 0,6 |
| 2-C16:0+2-C18:0/ (total C16:0+total C18:0) | | | 1,0 | 1,1 | | | 1,1 | 0,9 | 1,0 | 1,1 | | | 0,9 | 0,7 |

### Embodiments

The invention as discussed herein before can be detailed in various embodiments listed below:
1. Method for making an triacylglyceride composition comprising an interesterification step of a first fat comprising more than 55, 60, 65 or even 70 wt.% H-type fatty acids, drawn on the total amount of first fat, with a second fat to provide a triacylglyceride composition.
2. Method according to embodiment 1, wherein the first fat comprises more than 75 wt.% H-type fatty acids.
3. Method according to embodiment 1 or 2, wherein the first fat comprises more than 80 wt.% H-type fatty acids.
4. Method according to embodiments 1-3, wherein the first fat comprises more than 85 wt.% H-type fatty acids.
5. Method according to embodiments 1-4, wherein the first fat comprises more than 90 wt.% H-type fatty acids.
6. Method according to embodiments 1-5, wherein the first fat comprises more than 95 wt.% H-type fatty acids.
7. Method according to embodiments 1-6, wherein the first fat comprises more than 96 wt.% H-type fatty acids.
8. Method according to embodiments 1-7, wherein the first fat comprises more than 97 wt.% H-type fatty acids.
9. Method according to embodiments 1-8, wherein the first fat comprises more than 98 wt.% H-type fatty acids.
10. Method according to embodiments 1-9, wherein the first fat comprises an amount of saturated triacylglycerides of the H3 or StStSt type of at least 70 wt.%.
11. Method according to embodiments 1-10, wherein the first fat comprises an amount of saturated triacylglycerides of the H3 or StStSt type of at least 75 wt.%.
12. Method according to embodiments 1-11, wherein the first fat comprises an amount of saturated triacylglycerides of the H3 or StStSt type of at least 80 wt.%.
13. Method according to embodiment 1-12, wherein the first fat comprises an amount of saturated triacylglycerides of the H3 or StStSt type of at least 85 wt.%.
14. Method according to embodiment 1-13, wherein the first fat comprises an amount of saturated triacylglycerides of the H3 or StStSt type of at least 90wt.%.
15. Method according to embodiment 1-14, wherein the first fat comprises an amount of saturated triacylglycerides of the H3 or StStSt type of at least 95 wt.%.
16. Method according to embodiments 1-15, wherein the first fat comprises an amount of saturated triacylglycerides of the H3 or StStSt type of at least 96 wt.%.
17. Method according to embodiments 1-16, wherein the first fat comprises an amount of saturated triacylglycerides of the H3 or StStSt type of at least 97 wt.%.
18. Method according to embodiments 1-17, wherein the first fat comprises an amount of saturated triacylglycerides of the H3 or StStSt type of at least 98 wt.%.
19. Method according to embodiments 1-18, wherein the first fat comprises an amount of saturated triacylglycerides of the SSS or StStSt type of at least 70 wt.%.
20. Method according to embodiments 1-19, wherein the first fat comprises an amount of saturated triacylglycerides of the SSS or StStSt type of at least 75 wt.%.
21. Method according to embodiments 1-20, wherein the first fat comprises an amount of saturated triacylglycerides of the SSS or StStSt type of at least 80 wt.%.
22. Method according to embodiments 1-21, wherein the first fat comprises an amount of saturated triacylglycerides of the SSS or StStSt type of at least 85 wt.%.
23. Method according to embodiments 1-22, wherein the first fat comprises an amount of saturated triacylglycerides of the SSS or StStSt type of at least 90 wt.%.
24. Method according to embodiments 1-23, wherein the first fat comprises an amount of saturated triacylglycerides of the SSS or StStSt type of at least 95 wt.%.
25. Method according to embodiment 1-24, wherein the first fat comprises an amount of saturated triacylglycerides of the SSS or StStSt type of at least 96 wt.%.
26. Method according to embodiments 1-25, wherein the first fat comprises an amount of saturated triacylglycerides of the SSS or StStSt type of at least 97 wt.%.
27. Method according to embodiment 1-26, wherein the first fat comprises an amount of saturated triacylglycerides of the SSS or StStSt type of at least 98 wt.%.
28. Method according to embodiment 1-27, wherein the first fat comprises an amount of saturated triacylglycerides of the SSS or StStSt type of at least 99 wt.%.
29. Method according to embodiments 1-28, wherein the first fat comprises an amount of saturated fatty acids of at least 70 wt.%.
30. Method according to embodiments 1-29, wherein the first fat comprises an amount of saturated fatty acids of at least 75 wt.%.
31. Method according to embodiments 1-30, wherein the first fat comprises an amount of saturated fatty acids of at least 80 wt.%.
32. Method according to embodiments 1-31, wherein the first fat comprises an amount of saturated fatty acids of at least 85 wt.%.
33. Method according to embodiments 1-32, wherein the first fat comprises an amount of saturated fatty acids of at least 90 wt.%.
34. Method according to embodiments 1-33, wherein the first fat comprises an amount of saturated fatty acids of at least 95 wt.%.
35. Method according to embodiments 1-34, wherein the first fat comprises an amount of saturated fatty acids of at least 96 wt.%.
36. Method according to embodiments 1-35, wherein the first fat comprises an amount of saturated fatty acids of at least 97 wt.%.
37. Method according to embodiments 1-36, wherein the first fat comprises an amount of saturated fatty acids of at least 98 wt.%.
38. Method according to embodiments 1-37, wherein the first fat comprises an amount of saturated fatty acids of at least 99 wt.%.
39. Method according to embodiments 1-38, wherein the first fat comprises an amount of saturated fatty acids of the H-type of at least 70 wt.%.
40. Method according to embodiments 1-39, wherein the first fat comprises an amount of saturated fatty acids of the H-type of at least 75 wt.%.
41. Method according to embodiments 1-40, wherein the first fat comprises an amount of saturated fatty acids of the H-type of at least 80 wt.%.
42. Method according to embodiments 1-41, wherein the first fat comprises an amount of saturated fatty acids of the H-type of at least 85 wt.%.
43. Method according to embodiments 1-42, wherein the first fat comprises an amount of saturated fatty acids of the H-type of at least 90 wt.%.
44. Method according to embodiments 1-43, wherein the first fat comprises an amount of saturated fatty acids of the H-type of at least 95 wt.%.
45. Method according to embodiments 1-44, wherein the first fat comprises an amount of saturated fatty acids of the H-type of at least 96 wt.%.
46. Method according to embodiments 1-45, wherein the first fat comprises an amount of saturated fatty acids of the H-type of at least 97 wt.%.
47. Method according to embodiments 1-46, wherein the first fat comprises an amount of saturated fatty acids of the H-type of at least 98 wt.%.
48. Method according to embodiments 1-47, wherein the first fat comprises an amount of saturated fatty acids of the H-type of at least 99 wt.%.
49. Method according to embodiments 1-48, wherein the first fat comprises an amount of C18:0 of at least 55, 60, 65, or 70 wt.%.
50. Method according to embodiments 1-49, wherein the first fat comprises an amount of C18:0 of at least 75 wt.%.
51. Method according to embodiments 1-50, wherein the first fat comprises an amount of C18:0 of at least 80 wt.%.
52. Method according to embodiments 1-51, wherein the first fat comprises an amount of C18:0 of at least 85 wt.%.
53. Method according to embodiments 1-52, wherein the first fat comprises an amount of C18:0 of at least 90 wt.%.
54. Method according to embodiments 1-53, wherein the first fat comprises an amount of C18:0 of at least 95 wt.%.
55. Method according to embodiments 1-54, wherein the first fat comprises an amount of C18:0 of at least 96 wt.%.
56. Method according to embodiments 1-55, wherein the first fat comprises an amount of C18:0 of at least 97 wt.%.
57. Method according to embodiments 1-56, wherein the first fat comprises an amount of C18:0 of at least 98 wt.%.
58. Method according to embodiments 1-57, wherein the first fat comprises an amount of C18:0 of at least 99 wt.%.
59. Method according to embodiments 1-58, wherein the first fat comprises an amount of saturated fatty acids of the H-type at the 2-position of at least 55, 60, 65, or 70 wt.%.
60. Method according to embodiments 1-59, wherein the first fat comprises an amount of saturated fatty acids of the H-type at the 2-position of at least 75 wt.%.
61. Method according to embodiments 1-60, wherein the first fat comprises an amount of saturated fatty acids of the H-type at the 2-position of at least 80 wt.%.
62. Method according to embodiments 1-61, wherein the first fat comprises an amount of saturated fatty acids of the H-type at the 2-position of at least 85 wt.%.
63. Method according to embodiments 1-62, wherein the first fat comprises an amount of saturated fatty acids of the H-type at the 2-position of at least 90 wt.%.
64. Method according to embodiments 1-63, wherein the first fat comprises an amount of saturated fatty acids of the H-type at the 2-position of at least 95 wt.%.
65. Method according to embodiments 1-64, wherein the first fat comprises an amount of saturated fatty acids of the H-type at the 2-position of at least 96 wt.%.
66. Method according to embodiments 1-65, wherein the first fat comprises an amount of saturated fatty acids of the H-type at the 2-position of at least 97 wt.%.
67. Method according to embodiments 1-66, wherein the first fat comprises an amount of saturated fatty acids of the H-type at the 2-position of at least 98 wt.%.
68. Method according to embodiments 1-67, wherein the first fat comprises an amount of saturated fatty acids of the H-type at the 2-position of at least 99 wt.%.
69. Method according to embodiments 1-68, wherein the first fat comprises an amount of C18:0 at the 2-position of at least 70 wt.%.
70. Method according to embodiments 1-69, wherein the first fat comprises an amount of C18:0 at the 2-position of at least 75 wt.%.
71. Method according to embodiments 1-70, wherein the first fat comprises an amount of C18:0 at the 2-position of at least 80 wt.%.
72. Method according to embodiments 1-71, wherein the first fat comprises an amount of C18:0 at the 2-position of at least 85 wt.%.
73. Method according to embodiments 1-72, wherein the first fat comprises an amount of C18:0 at the 2-position of at least 90 wt.%.
74. Method according to embodiments 1-73, wherein the first fat comprises an amount of C18:0 at the 2-position of at least 95 wt.%.
75. Method according to embodiments 1-74, wherein the first fat comprises an amount of C18:0 at the 2-position of at least 96 wt.%.
76. Method according to embodiments 1-75, wherein the first fat comprises an amount of C18:0 at the 2-position of at least 97 wt.%.
77. Method according to embodiments 1-76, wherein the first fat comprises an amount of C18:0 at the 2-position of at least 98 wt.%.
78. Method according to embodiments 1-77, wherein the first fat comprises an amount of C18:0 at the 2-position of at least 99 wt.%.
79. Method according to embodiments 1-78, wherein the first fat comprises an amount of C16:0, preferably at the 2-position, is at most 11 wt.%.
80. Method according to embodiments 1-79, wherein the first fat comprises an amount of C16:0, , preferably at the 2-position of at most 10 wt.%.
81. Method according to embodiments 1-80, wherein the first fat comprises an amount of C16:0, preferably at the 2-position of at most 9 wt.%.
82. Method according to embodiments 1-81, wherein the first fat comprises an amount of C16:0, preferably at the 2-position of at most 8 wt.%.
83. Method according to embodiments 1-82, wherein the first fat comprises an amount of C16:0, preferably at the 2-position of at most 7 wt.%.
84. Method according to embodiments 1-83, wherein the first fat comprises an amount of C16:0, preferably at the 2-position of at most 6 wt.%.
85. Method according to embodiments 1-84, wherein the first fat comprises an amount of C16:0, preferably at the 2-position of at most 5 wt.%.
86. Method according to embodiments 1-85, wherein the first fat comprises an amount of C16:0, preferably at the 2-position of at most 4 wt.%.
87. Method according to embodiments 1-86, wherein the first fat comprises an amount of C16:0, preferably at the 2-position of at most 3 wt.%.
88. Method according to embodiments 1-87, wherein the first fat comprises an amount of C16:0, , preferably at the 2-position of at most 2 wt.%.
89. Method according to embodiments 1-88, wherein the first fat comprises an amount of C16:0, preferably at the 2-position of at most 1 wt.%.
90. Method according to embodiments 1-89, wherein the first fat comprises a ratio of C18:0/C16:0 at the 2-position from 50 to 99.
91. Method according to embodiments 1-90, wherein the first fat comprises a ratio of C18:0/C16:0 at the 2-position from 55-95.
92. Method according to embodiments 1-91, wherein the first fat comprises a ratio of C18:0/C16:0 at the 2-position from 60-90.
93. Method according to embodiments 1-92, wherein the first fat comprises a ratio of C18:0/C16:0 at the 2-position from 65-85.
94. Method according to embodiments 1-93, wherein the first fat comprises a ratio of C18:0/C16:0 at the 2-position from 70-80.
95. Method according to embodiments 1-94, wherein the first fat is non-hydrogenated.
96. Method according to embodiments 1-95, wherein the first fat does not contain (partly) hydrogenated triglycerides.
97. Method according to embodiments 1-96, wherein the first fat is an edible oil or fat preferably not based on, or containing, palm oil or palm oil-based or palm oil-derived fractions.
98. Method according to embodiments 1-97, wherein the second fat is an edible oil or fat, preferably selected from the group consisting of coconut oil, sunflower oil, rapeseed oil, shea and fractions thereof, linseed, palm oil and fractions thereof and palm kernel and fractions thereof, and mixtures thereof.
99. Method according to embodiments 1-98, wherein the second fat is selected from the group consisting of coconut oil, sunflower oil, rapeseed oil, shea and fractions thereof, linseed, and mixtures thereof.
100. Method according to embodiments 1-99, wherein the second fat is not based on, or containing, palm oil or palm oil-based or palm oil-derived fractions.
101. Method according to embodiments 1-100, wherein the second fat does not contain (partly) hydrogenated triglycerides.
102. Method according to embodiments 1-101, wherein the interesterification step is a chemical interesterification.
103. Method according to embodiments 1-102, wherein the interesterification step is an enzymatic interesterification.
104. Method according to embodiments 1-103, wherein the chemical interesterification is a catalytic interesterification.
105. Method according to embodiments 1-104, wherein the amount of the first fat drawn on the total fat is from 10 to 65 wt.%.
106. Method according to embodiments 1-105, wherein the amount of the first fat drawn on the total fat is from 15 to 25 wt.%.
107. Method according to embodiments 1-106, wherein the amount of the first fat drawn on the total fat is from 25 to 35 wt.%.
108. Method according to embodiments 1-107, wherein the amount of the first fat drawn on the total fat is from 30 to 45 wt.%.
109. Method according to embodiments 1-108, wherein the amount of the first fat drawn on the total fat is from 35 to 50 wt.%.
110. Method according to embodiments 1-109, wherein the amount of the first fat drawn on the total fat is from 40 to 55 wt.%.
111. Method according to embodiments 1-110, wherein the amount of the first fat drawn on the total fat is from 45 to 60 wt.%.
112. Method according to embodiments 1-111, wherein the amount of the first fat drawn on the total fat is from 50 to 65 wt.%.
113. Method according to embodiments 1-112, wherein the first and second fat are blended in a ratio (first fat/second fat) from 95/5 to 5/95.
114. Method according to embodiments 1-113, wherein the first and second fat are blended in a ratio (first fat/second fat) from 90/10 to 10/90.
115. Method according to embodiments 1-114, wherein the first and second fat are blended in a ratio (first fat/second fat) from 85/15 to 15/85.
116. Method according to embodiments 1-115, wherein the first and second fat are blended in a ratio (first fat/second fat) from 80/20 to 20/80.
117. Method according to embodiments 1-116, wherein the first and second fat are blended in a ratio (first fat/second fat) from 75/25 to 25/75.
118. Method according to embodiments 1-117, wherein the first and second fat are blended in a ratio (first fat/second fat) from 70/30 to 30/70.
119. Method according to embodiments 1-118, wherein the first and second fat are blended in a ratio (first fat/second fat) from 65/35 to 35/65.
120. Method according to embodiments 1-119, wherein the first and second fat are blended in a ratio (first fat/second fat) from 60/40 to 40/60.
121. Method according to embodiments 1-120, wherein the first and second fat are blended into a fat blend and subjected to a neutralisation and/or deodorisation step prior to and/or following interesterification.
122. Method according to embodiments 1-121, wherein the triacylglyceride composition comprises an amount of triacylglycerides of the H3 or StStSt type of at least 40 wt.% drawn on the total amount of the triacylglyceride composition.
123. Method according to embodiments 1-122, wherein the triacylglyceride composition comprises an amount of triacylglycerides of the H3 or StStSt type of at least 45 wt.% drawn on the total amount of the triacylglyceride composition.
124. Method according to embodiments 1-123, wherein the triacylglyceride composition comprises an amount of triacylglycerides of the H3 or StStSt type of at least 50 wt.% drawn on the total amount of the triacylglyceride composition.
125. Method according to embodiments 1-124 wherein the triacylglyceride composition comprises an amount of triacylglycerides of the H3 or StStSt type of at least 55 wt.% drawn on the total amount of the triacylglyceride composition.
126. Method according to embodiments 1-125, wherein the triacylglyceride composition comprises an amount of triacylglycerides of the H3 or StStSt type of at least 60 wt.% drawn on the total amount of the triacylglyceride composition.
127. Method according to embodiments 1-126, wherein the triacylglyceride composition comprises an amount of triacylglycerides of the H3 or StStSt type of at least 65 wt.% drawn on the total amount of the triacylglyceride composition.
128. Method according to embodiments 1-127, wherein the triacylglyceride composition comprises an amount of triacylglycerides of the H3 or StStSt type of at least 70 wt.% drawn on the total amount of the triacylglyceride composition.
129. Method according to embodiments 1-128, wherein the triacylglyceride composition comprises an amount of triacylglycerides of the SSS or StStSt type of at least 40 wt.% drawn on the total amount of the triacylglyceride composition.
130. Method according to embodiments 1-129, wherein the triacylglyceride composition comprises an amount of triacylglycerides of the SSS or StStSt type of at least 45 wt.% drawn on the total amount of the triacylglyceride composition.
131. Method according to embodiments 1-130, wherein the triacylglyceride composition comprises an amount of triacylglycerides of the SSS or StStSt type of at least 50 wt.% drawn on the total amount of the triacylglyceride composition.
132. Method according to embodiments 1-131, wherein the triacylglyceride composition comprises an amount of triacylglycerides of the SSS or StStSt type of at least 55 wt.% drawn on the total amount of the triacylglyceride composition.
133. Method according to embodiments 1-132, wherein the triacylglyceride composition comprises an amount of triacylglycerides of the SSS or StStSt type of at least 60 wt.% drawn on the total amount of the triacylglyceride composition.
134. Method according to embodiments 1-133, wherein the triacylglyceride composition comprises an amount of triacylglycerides of the SSS or StStSt type of at least 65 wt.% drawn on the total amount of the triacylglyceride composition.
135. Method according to embodiments 1-134, wherein the triacylglyceride composition comprises an amount of triacylglycerides of the SSS or StStSt type of at least 70 wt.% drawn on the total amount of the triacylglyceride composition.
136. Method according to embodiments 1-135, wherein the triacylglyceride composition comprises an amount of saturated fatty acids of at least 65 wt.% drawn on the total amount of fatty acids in the triacylglyceride composition.
137. Method according to embodiments 1-136, wherein the triacylglyceride composition comprises an amount of saturated fatty acids of at least 70 wt.% drawn on the total amount of fatty acids in the triacylglyceride composition.
138. Method according to embodiments 1-137, wherein the triacylglyceride composition comprises an amount of saturated fatty acids of at least 71 wt.% drawn on the total amount of fatty acids in the triacylglyceride composition.
139. Method according to embodiments 1-138, wherein the triacylglyceride composition comprises an amount of saturated fatty acids of at least 72 wt.% drawn on the total amount of fatty acids in the triacylglyceride composition.
140. Method according to embodiments 1-139, wherein the triacylglyceride composition comprises an amount of saturated fatty acids of at least 73 wt.% drawn on the total amount of fatty acids in the triacylglyceride composition.
141. Method according to embodiments 1-140, wherein the triacylglyceride composition comprises an amount of saturated fatty acids of at least 74 wt.% drawn on the total amount of fatty acids in the triacylglyceride composition.
142. Method according to embodiments 1-141, wherein the triacylglyceride composition comprises an amount of saturated fatty acids of at least 75 wt.% drawn on the total amount of fatty acids in the triacylglyceride composition.
143. Method according to embodiments 1-142, wherein the triacylglyceride composition comprises an amount of saturated fatty acids of at least 80 wt.% drawn on the total amount of fatty acids in the triacylglyceride composition.
144. Method according to embodiments 1-143, wherein the triacylglyceride composition comprises an amount of saturated fatty acids of at least 85 wt.% drawn on the total amount of fatty acids in the triacylglyceride composition.
145. Method according to embodiments 1-144, wherein the triacylglyceride composition comprises an amount of from 20 to 50 wt.% of saturated fatty acids of the H-type drawn on the total amount of fatty acids in the triacylglyceride composition.
146. Method according to embodiments 1-145, wherein the triacylglyceride composition comprises an amount of from 25 to 45 wt.% of saturated fatty acids of the H-type drawn on the total amount of fatty acids in the triacylglyceride composition.
147. Method according to embodiments 1-146, wherein the triacylglyceride composition comprises an amount of from 27 to 42 wt.% of saturated fatty acids of the H-type drawn on the total amount of fatty acids in the triacylglyceride composition.
148. Method according to embodiments 1-147, wherein the triacylglyceride composition comprises from 20 to 45 wt.% of C18:0.
149. Method according to embodiments 1-148, wherein the triacylglyceride composition comprises from 22 to 40 wt.% of C18:0.
150. Method according to embodiments 1-149, wherein the triacylglyceride composition comprises from 24 to 35 wt.% of C18:0.
151. Method according to embodiments 1-150, wherein the triacylglyceride composition comprises an amount of C16:0 of at most 10 wt.%.
152. Method according to embodiments 1-151, wherein the triacylglyceride composition comprises an amount of C16:0 of at most 9 wt.%.
153. Method according to embodiments 1-152, wherein the triacylglyceride composition comprises an amount of C16:0 of at most 8 wt.%.
154. Method according to embodiments 1-153, wherein the triacylglyceride composition comprises an amount of C16:0 of at most 7 wt.%.
155. Method according to embodiments 1-154, wherein the triacylglyceride composition comprises an amount of C16:0 of at most 6 wt.%.
156. Method according to embodiments 1-155, wherein the triacylglyceride composition comprises an amount of C16:0 of at most 5 wt.%.
157. Method according to embodiments 1-156, wherein the triacylglyceride composition comprises an amount of C16:0 of between 5 and 15 wt.%.
158. Method according to embodiments 1-157, wherein the triacylglyceride composition comprises an amount of C16:0 of between 6 and 12 wt.%.
159. Method according to embodiments 1-158, wherein the triacylglyceride composition comprises an amount of C16:0 of between 7 and 10 wt.%.
160. Method according to embodiments 1-159, wherein the triacylglyceride composition comprises an amount of saturated fatty acids of the H-type at the 2-position is between 20 and 50 wt.%
161. Method according to embodiments 1-160, wherein the triacylglyceride composition comprises an amount of saturated fatty acids of the H-type at the 2-position is between 25 and 45 wt.%.
162. Method according to embodiments 1-161, wherein the triacylglyceride composition comprises an amount of saturated fatty acids of the H-type at the 2-position is between 30 and 40 wt.%.
163. Method according to embodiments 1-162, wherein the triacylglyceride composition comprises an amount of saturated fatty acids of C18:0 at the 2-position is between 15 and 40 wt.%.
164. Method according to embodiments 1-163, wherein the triacylglyceride composition comprises an amount of saturated fatty acids of C18:0 at the 2-position is between 20 and 38 wt.%
165. Method according to embodiments 1-164, wherein the triacylglyceride composition comprises an amount of saturated fatty acids of C18:0 at the 2-position is between 22 and 35 wt.%.
166. Method according to embodiments 1-165, wherein the triacylglyceride composition comprises an amount of C16:0 at the 2-position is between 5 and 12 wt.%.
167. Method according to embodiments 1-166, wherein the triacylglyceride composition comprises an amount of C16:0 at the 2-position is between 6 and 11 wt.%.
168. Method according to embodiments 1-167, wherein the triacylglyceride composition comprises an amount of C16:0 at the 2-position is between 7 and 10 wt.%.
169. Method according to embodiments 1-168, wherein the triacylglyceride composition has a ratio of C18:0/C16:0 at the 2-position from 1 to 10.
170. Method according to embodiments 1-169, wherein the triacylglyceride composition has a ratio of C18:0/C16:0 at the 2-position from 1.5 to 9.
171. Method according to embodiments 1-170, wherein the triacylglyceride composition has a ratio of C18:0/C16:0 at the 2-position from 2 to 8.
172. Method according to embodiments 1-171, wherein the triacylglyceride composition has a ratio of C18:0 at the 2-position to a total C18:0 from 0.8 to 1.5.
173. Method according to embodiments 1-172, wherein the triacylglyceride composition has a ratio of C18:0 at the 2-position to a total C18:0 from 0.85 to 1.45.
174. Method according to embodiments 1-173, wherein the triacylglyceride composition has a ratio of C18:0 at the 2-position to a total C18:0 from 0.9 to 1.4.
175. Method according to embodiments 1-174, wherein the triacylglyceride composition has a ratio of C18:0 at the 2-position to a total C18:0 from 0.95 to 1.35.
176. Method according to embodiments 1-175, wherein the triacylglyceride composition has a ratio of C18:0 at the 2-position to a total C18:0 from 1 to 1.25.
177. Method according to embodiments 1-176, wherein the triacylglyceride composition has a ratio of C18:0 at the 2-position to a total C18:0 from 1.05 to 1.2.
178. Method according to embodiments 1-177, wherein the triacylglyceride composition has a ratio of C18:0 at the 2-position to a total U of more than 0.5.
179. Method according to embodiments 1-178, wherein the triacylglyceride composition has a ratio of C18:0 at the 2-position to a total U at the 2-position from 0.80 to 2.
180. Method according to embodiments 1-179, wherein the triacylglyceride composition has a ratio of C18:0 at the 2-position to a total U at the 2-position from 0.85 to 1.95.
181. Method according to embodiments 1-180, wherein the triacylglyceride composition has a ratio of C18:0 at the 2-position to a total U at the 2-position from 0.90 to 1.9.
182. Method according to embodiments 1-181, wherein the triacylglyceride composition has a ratio of C18:0 at the 2-position to a total U at the 2-position from 0.95 to 1.85.
183. Method according to embodiments 1-182, wherein the triacylglyceride composition has a ratio of C18:0 at the 2-position to a total U at the 2-position from 1 to 2.
184. Method according to embodiments 1-183, wherein the triacylglyceride composition has a ratio of C18:0 at the 2-position to a total U at the 2-position from 1.05 to 2.
185. Method according to embodiments 1-184, wherein the triacylglyceride composition has a ratio of C16:0 at the 2-position to a total C16:0+ C18:0 at the 2-position of less than 0.5.
186. Method according to embodiments 1-185, wherein the triacylglyceride composition has a ratio of C16:0 at the 2-position to a total C16:0+ C18:0 at the 2-position from 0.02 to 0.75.
187. Method according to embodiments 1-186, wherein the triacylglyceride composition has a ratio of C16:0 at the 2-position to a total C16:0+ C18:0 at the 2-position from 0.03 to 0.70.
188. Method according to embodiments 1-187, wherein the triacylglyceride composition has a ratio of C16:0 at the 2-position to a total C16:0+ C18:0 at the 2-position from 0.05 to 0.65.
189. Method according to embodiments 1-188, wherein the triacylglyceride composition has a ratio of C16:0 at the 2-position to a total C16:0+ C18:0 at the 2-position from 0.07 to 0.60.
190. Method according to embodiments 1-189, wherein the triacylglyceride composition has a ratio of C16:0 at the 2-position to a total C16:0+ C18:0 at the 2-position from 0.09 to 0.65.
191. Method according to embodiments 1-190, wherein the triacylglyceride composition has a ratio of C16:0 at the 2-position to a total C16:0+ C18:0 at the 2-position from 0.10 to 0.55.
192. Triacylglyceride composition (hard stock) obtainable by the method of embodiments 1-191.
193. Triacylglyceride composition comprising fatty acids, in wt.% on the total amount of fatty acids, wherein the triglyceride composition is an interesterified fat and characterised in having:
- optionally, C12:0 from 15 to 40 wt.%, preferably from 20 to 35 wt.%, more preferably from 22 to 25 wt.%;
- optionally, C14:0 from 5 to 15 wt.%, preferably from 7 to 12 wt.%, more preferably from 7 to 10 wt.%;
- optionally, C12:0 and C14:0 from 10 to 40 wt.%, preferably from 15 to 35 wt.%, more preferably from 20 to 30 wt.%;
- optionally, C16:0 from 5 to 15 wt.%, preferably from 6 to 12 wt.%, more preferably from 7 to 10 wt.%;
- optionally, C18:0 between 15 and 45 wt.%, preferably from 20 to 40 wt.%, more preferably from 24 to 35 wt.%;
- optionally, C16:0 and C18:0 25 to 60 wt.%, preferably from 27 to 55 wt.%, more preferably from 32 to 50 wt.%;
- optionally, C18:1 of less than 20 wt.%, preferably from 5 to 20 wt.%, more preferably from 8 to 18 wt.%, even more preferably from 10 to 15 wt.%;
- optionally, a total amount of fully saturated fatty acids from 65 to 80 wt.%, preferably from 68 to 78 wt.%, more preferably from 70 to 75 wt.%;
- optionally, an amount of poly unsaturated fatty acids (PUFA) of less than 25 wt.%, preferably from 10 to 20 wt.%, more preferably from 12 to 18 wt.%, even more preferably from 13 to 17 wt.%;
- optionally, less than 0.5 wt.% trans fatty acids, preferably less than 0.2 wt.%, more preferably less than 0.1 wt.%.
194. Triacylglyceride composition according to embodiment 193, comprising fatty acids on the 2-position, in wt.% on the total amount of fatty acids on the 2-position, the triglyceride composition is an interesterified fat and is characterised in :
- optionally, C12:0 from 10 to 40 wt.%, preferably from 15 to 35 wt.%, more preferably from 20 to 30 wt.%;
- optionally, C14:0 from 7 to 13 wt.%, preferably from 8 to 12 wt.%, more preferably from 9 to 11 wt.%;
- optionally, C12:0 and/or C14 from 17 to 55 wt.%, preferably from 20 to 40 wt.%, more preferably from 25 to 35 wt.%;
- optionally, C16:0 from 5 to 12 wt.%, preferably from 6 to 11 wt.%, more preferably from 7 to 9 wt.%;
- optionally, C18:0 from 10 to 60 wt.%, preferably from 15 to 50 wt.%, more preferably from 20 to 45 wt.%;
- optionally, C16:0 and C18:0 from 30 to 45 wt.%, preferably from 32 to 40 wt.%;
- optionally, C18:1 from 5 to 15 wt.%;
- optionally, C18:2 and C18:3 from 5 to 15 wt.%;
- optionally, a total amount of saturated fatty acids from 60 to 90 wt.%, preferably from 65 to 85 wt.%, more preferably from 70 to 80 wt.%;
- optionally, an amount of poly unsaturated fatty acids (PUFA) of less than 25 wt.%, preferably from 10 to 20 wt.%, more preferably from 12 to 18 wt.%, even more preferably from 13 to 17 wt.%;
- optionally, less than 0.5 wt.% trans fatty acids wt.%, preferably less than 0.2 wt.%, more preferably less than 0.1 wt.%.
195. Triacylglyceride composition according to embodiments 192-194, wherein in the triglyceride composition has an SFC profile of

| Temperature | % | % |
|---|---|---|
| 5 °C | 75-85 | 73-83 |
| 10 °C | 65-80 | 68-78 |
| 20 °C | 40-60 | 44-53 |
| 30 °C | 15-30 | 17-27 |
| 35 °C | 5-20 | 6-16 |

196. Triacylglyceride composition according to embodiments 192-194, wherein in the triglyceride composition has an SFC profile of

| Temperature | % | % |
|---|---|---|
| 5 °C | 60-80 | 62-72 |
| 10 °C | 55-70 | 56-66 |
| 20 °C | 25-40 | 28-38 |
| 30 °C | 5-15 | 5-15 |
| 35 °C | 1-6 | 1-10 |

197. Triacylglyceride composition according to embodiments 192- 196, comprising an amount of triacylglycerides of the H3 or StStSt type of at least 40 wt.% drawn on the total amount of the triacylglyceride composition.
198. Triacylglyceride composition according to embodiments 192- 197, comprising an amount of triacylglycerides of the H3 or StStSt type of at least 45 wt.% drawn on the total amount of the triacylglyceride composition.
199. Triacylglyceride composition according to embodiments 192- 198, comprising an amount of triacylglycerides of the H3 or StStSt type of at least 50 wt.% drawn on the total amount of the triacylglyceride composition.
200. Triacylglyceride composition according to embodiments 192- 199, comprising an amount of triacylglycerides of the H3 or StStSt type of at least 55 wt.% drawn on the total amount of the triacylglyceride composition.
201. Triacylglyceride composition according to embodiments 192- 200, comprising an amount of triacylglycerides of the H3 or StStSt type of at least 60 wt.% drawn on the total amount of the triacylglyceride composition.
202. Triacylglyceride composition according to embodiments 192- 201, comprising an amount of triacylglycerides of the H3 or StStSt type of at least 65 wt.% drawn on the total amount of the triacylglyceride composition.
203. Triacylglyceride composition according to embodiments 192- 202, comprising an amount of triacylglycerides of the H3 or StStSt type of at least 70 wt.% drawn on the total amount of the triacylglyceride composition.
204. Triacylglyceride composition according to embodiments 192- 203, comprising an amount of triacylglycerides of the SSS or StStSt type of at least 40 wt.% drawn on the total amount of the triacylglyceride composition.
205. Triacylglyceride composition according to embodiments 192- 204, comprising an amount of triacylglycerides of the SSS or StStSt type of at least 45 wt.% drawn on the total amount of the triacylglyceride composition.
206. Triacylglyceride composition according to embodiments 192- 205, comprising an amount of triacylglycerides of the SSS or StStSt type of at least 50 wt.% drawn on the total amount of the triacylglyceride composition.
207. Triacylglyceride composition according to embodiments 192- 206, comprising an amount of triacylglycerides of the SSS or StStSt type of at least 55 wt.% drawn on the total amount of the triacylglyceride composition.
208. Triacylglyceride composition according to embodiments 192- 207, comprising an amount of triacylglycerides of the SSS or StStSt type of at least 60 wt.% drawn on the total amount of the triacylglyceride composition.
209. Triacylglyceride composition according to embodiments 192- 207, comprising an amount of triacylglycerides of the SSS or StStSt type of at least 65 wt.% drawn on the total amount of the triacylglyceride composition.
210. Triacylglyceride composition according to embodiments 192- 209, comprising an amount of triacylglycerides of the SSS or StStSt type of at least 70 wt.% drawn on the total amount of the triacylglyceride composition.
211. Triacylglyceride composition according to embodiments 192- 210, comprising an amount of saturated fatty acids of at least 65 wt.% drawn on the total amount of fatty acids in the triacylglyceride composition.
212. Triacylglyceride composition according to embodiments 192- 211, comprising an amount of saturated fatty acids of at least 70 wt.% drawn on the total amount of fatty acids in the triacylglyceride composition.
213. Triacylglyceride composition according to embodiments 192- 212, comprising an amount of saturated fatty acids of at least 71 wt.% drawn on the total amount of fatty acids in the triacylglyceride composition.
214. Triacylglyceride composition according to embodiments 192- 213, comprising an amount of saturated fatty acids of at least 72 wt.% drawn on the total amount of fatty acids in the triacylglyceride composition.
215. Triacylglyceride composition according to embodiments 192- 214, comprising an amount of saturated fatty acids of at least 73 wt.% drawn on the total amount of fatty acids in the triacylglyceride composition.
216. Triacylglyceride composition according to embodiments 192- 215, comprising an amount of saturated fatty acids of at least 74 wt.% drawn on the total amount of fatty acids in the triacylglyceride composition.
217. Triacylglyceride composition according to embodiments 192- 216, comprising an amount of saturated fatty acids of at least 75 wt.% drawn on the total amount of fatty acids in the triacylglyceride composition.
218. Triacylglyceride composition according to embodiments 192- 217, comprising an amount of saturated fatty acids of at least 80 wt.% drawn on the total amount of fatty acids in the triacylglyceride composition.
219. Triacylglyceride composition according to embodiments 192- 218, comprising an amount of saturated fatty acids of at least 85 wt.% drawn on the total amount of fatty acids in the triacylglyceride composition.
220. Triacylglyceride composition according to embodiments 192- 219, comprising an amount of from 20 to 60 wt.% of saturated fatty acids of the H-type drawn on the total amount of fatty acids in the triacylglyceride composition.
221. Triacylglyceride composition according to embodiments 192- 220, comprising an amount of from 25 to 50 wt.% of saturated fatty acids of the H-type drawn on the total amount of fatty acids in the triacylglyceride composition.
222. Triacylglyceride composition according to embodiments 192- 221, comprising an amount of from 28 to 45 wt.% of saturated fatty acids of the H-type drawn on the total amount of fatty acids in the triacylglyceride composition.
223. Triacylglyceride composition according to embodiments 192- 222, comprising from 20 to 45 wt.% of C18:0.
224. Triacylglyceride composition according to embodiments 192- 223, comprising from 22 to 40 wt.% of C18:0.
225. Triacylglyceride composition according to embodiments 192- 224, comprising from 24 to 35 wt.% of C18:0.
226. Triacylglyceride composition according to embodiments 192- 225, comprising an amount of C16:0 of at most 10 wt.%.
227. Triacylglyceride composition according to embodiments 192- 226, comprising an amount of C16:0 of at most 9 wt.%.
228. Triacylglyceride composition according to embodiments 192- 227, comprising an amount of C16:0 of at most 8 wt.%.
229. Triacylglyceride composition according to embodiments 192- 228, comprising an amount of C16:0 of at most 7 wt.%.
230. Triacylglyceride composition according to embodiments 192- 229, comprising an amount of C16:0 of at most 6 wt.%.
231. Triacylglyceride composition according to embodiments 192- 230, comprising an amount of C16:0 of at most 5 wt.%.
232. Triacylglyceride composition according to embodiments 192- 231, comprising amount of C16:0 of between 5 and 15 wt.%.
233. Triacylglyceride composition according to embodiments 192- 232, comprising amount of C16:0 of between 6 and 12 wt.%.
234. Triacylglyceride composition according to embodiments 192- 233, comprising amount of C16:0 of between 7 and 10 wt.%.
235. Triacylglyceride composition according to embodiments 192- 234, comprising an amount of saturated fatty acids of the H-type at the 2-position is between 20 and 50 wt.%
236. Triacylglyceride composition according to embodiments 192- 235, comprising an amount of saturated fatty acids of the H-type at the 2-position is between 25 and 45 wt.% wt.%.
237. Triacylglyceride composition according to embodiments 192- 236, comprising an amount of saturated fatty acids of the H-type at the 2-position is between 30 and 40 wt.%.
238. Triacylglyceride composition according to embodiments 192- 237, comprising an amount of saturated fatty acids of C18:0 at the 2-position is between 15 and 40 wt.%.
239. Triacylglyceride composition according to embodiments 192- 238, comprising an amount of saturated fatty acids of C18:0 at the 2-position is between 20 and 38 wt.%
240. Triacylglyceride composition according to embodiments 192- 239, comprising an amount of saturated fatty acids of C18:0 at the 2-position is between 25 and 35 wt.%.
241. Triacylglyceride composition according to embodiments 192- 240, comprising an amount of C16:0 at the 2-position is between 5 and 12 wt.%.
242. Triacylglyceride composition according to embodiments 192- 241, comprising an amount of C16:0 at the 2-position is between 6 and 11 wt.%.
243. Triacylglyceride composition according to embodiments 192- 242, comprising an amount of C16:0 at the 2-position is between 7 and 10 wt.%.
244. Triacylglyceride composition according to embodiments 192- 243, comprising a ratio of C18:0/C16:0 at the 2-position from 1 to 10.
245. Triacylglyceride composition according to embodiments 192- 244, comprising a ratio of C18:0/C16:0 at the 2-position from 2 to 9.
246. Triacylglyceride composition according to embodiments 192- 245, comprising a ratio of C18:0/C16:0 at the 2-position from 3 to 8.
247. Triacylglyceride composition according to embodiments 192- 246, comprising a ratio of C18:0 at the 2-position to a total C18:0 from 0.8 to 1.5.
248. Triacylglyceride composition according to embodiments 192- 247, comprising a ratio of C18:0 at the 2-position to a total C18:0 from 0.85 to 1.45.
249. Triacylglyceride composition according to embodiments 192- 248, comprising a ratio of C18:0 at the 2-position to a total C18:0 from 0.9 to 1.4.
250. Triacylglyceride composition according to embodiments 192- 249, comprising a ratio of C18:0 at the 2-position to a total C18:0 from 0.95 to 1.35.
251. Triacylglyceride composition according to embodiments 192- 250, comprising a ratio of C18:0 at the 2-position to a total C18:0 from 1 to 1.25.
252. Triacylglyceride composition according to embodiments 192- 251, comprising a ratio of C18:0 at the 2-position to a total C18:0 from 1.05 to 1.2.
253. Triacylglyceride composition according to embodiments 192- 252, comprising a ratio of C18:0 at the 2-position to a total U of more than 0.5.
254. Triacylglyceride composition according to embodiments 192- 253, comprising a ratio of C18:0 at the 2-position to a total U at the 2-position from 0.80 to 2.
255. Triacylglyceride composition according to embodiments 192- 254, comprising a ratio of C18:0 at the 2-position to a total U at the 2-position from 0.85 to 1.95.
256. Triacylglyceride composition according to embodiments 192- 255, comprising a ratio of C18:0 at the 2-position to a total U at the 2-position from 0.90 to 1.9.
257. Triacylglyceride composition according to embodiments 192- 256, comprising a ratio of C18:0 at the 2-position to a total U at the 2-position from 0.95 to 1.85.
258. Triacylglyceride composition according to embodiments 192- 257, comprising a ratio of C18:0 at the 2-position to a total U at the 2-position from 1 to 2.
259. Triacylglyceride composition according to embodiments 192- 258, comprising a ratio of C18:0 at the 2-position to a total U at the 2-position from 1.05 to 2.
260. Triacylglyceride composition according to embodiments 192- 259, comprising a ratio of C16:0 at the 2-position to a total C16:0+ C18:0 at the 2-position of less than 0.5.
261. Triacylglyceride composition according to embodiments 192- 260, comprising a ratio of C16:0 at the 2-position to a total C16:0+ C18:0 at the 2-position from 0.02 to 0.75.
262. Triacylglyceride composition according to embodiments 192- 261, comprising a ratio of C16:0 at the 2-position to a total C16:0+ C18:0 at the 2-position from 0.03 to 0.70.
263. Triacylglyceride composition according to embodiments 192- 262, comprising a ratio of C16:0 at the 2-position to a total C16:0+ C18:0 at the 2-position from 0.05 to 0.65.
264. Triacylglyceride composition according to embodiments 192- 263, comprising a ratio of C16:0 at the 2-position to a total C16:0+ C18:0 at the 2-position from 0.07 to 0.60.
265. Triacylglyceride composition according to embodiments 192- 264, comprising a ratio of C16:0 at the 2-position to a total C16:0+ C18:0 at the 2-position from 0.09 to 0.65.
266. Triacylglyceride composition according to embodiments 192- 265, comprising a ratio of C16:0 at the 2-position to a total C16:0+ C18:0 at the 2-position from 0.10 to 0.55.
267. Hard stock or structuring fat comprising a triacylglyceride composition as defined in any of the previous embodiments.
268. Use of a triacylglyceride composition as defined in any of the previous embodiments as a hard stock or structuring fat.
269. Use of a triacylglyceride composition according to embodiment 174 in the preparation of a water-continuous or fat-continuous edible emulsion.
270. Method for the preparation of a water-continuous or fat-continuous edible emulsion such as a margarine or spread comprising the steps of combining a hard stock as defined in any of the previous embodiments, a liquid oil and an aqueous phase.
271. Margarine, optionally obtainable by the method of embodiment 176, comprising a triglyceride composition as defined in any of the previous embodiments.
272. Spread, optionally obtainable by the method of embodiment 176, comprising a triglyceride composition as defined in any of the previous embodiments.

## Claims

1. Method for making a triacylglyceride composition comprising an interesterification step of a first fat comprising more than 55 wt.% H-type fatty acids, drawn on the total amount of first fat, with a second fat to provide a triacylglyceride composition, the triacylglyceride composition
- comprising an amount of saturated fatty acids of the H-type at the 2-position of at between 20 and 50 wt.% calculated on the total amount of fatty acids at the 2-position, and
- having C18:1 of less than 20 wt.%,
wherein the first and second fat are non-hydrogenated triglycerides and are not based on, or containing, palm oil or palm oil-based or palm oil-derived fractions, and wherein H is C16:0 (palmitic acid) and/or C18:0 (stearic acid).

2. Method according to claim 1, wherein the first fat comprises an amount of C18:0 at the 2-position of at least 55 wt.% and/or an amount of C16:0 at the 2-position of at most 11 wt.%.

3. Method according to claim 1 or 2, wherein the interesterification step is a random interesterification.

4. Method according to any of claims 1 - 3, wherein the amount of the first fat drawn on the total fat is from 10 to 65 wt.%.

5. Method according to any of claims 1 - 4, wherein the triacylglyceride composition comprises an amount of C16:0 of between 5 and 15 wt.%.

6. Method according to any of claims 1 - 5, wherein the triacylglyceride composition comprises an amount of triacylglycerides of the H3 or StStSt type of at least 40 wt.% drawn on the total amount of the triacylglyceride composition.

7. Interesterified triacylglyceride composition (hard stock) obtainable by the method of any of claims 1 - 6.

8. Interesterified triacylglyceride composition comprising fatty acids, in wt.% on the total amount of fatty acids, wherein the triglyceride composition is an interesterified fat comprising an amount of saturated fatty acids of the H-type at the 2-position of between 20 and 50 wt.%, calculated on the total amount of fatty acids at the 2-position, and further **characterised in** having:
- the sum of C16:0 and C18:0 from 25 to 60 wt.%;
- C18:1 of less than 20 wt.%;
- less than 0.5 wt.% trans fatty acids%.

9. Triacylglyceride composition according to claim 8, wherein the triglyceride composition is an interesterified fat and **characterised in** having fatty acids at the 2-position:
- the sum of C16:0 and C18:0 from 30 to 45 wt.%;
- C18:1 from 5 to 15 wt.%;
- less than 0.5 wt.% trans fatty acids wt.%;
in wt.% on the total amount of fatty acids on the 2-position.

10. Hard stock or structuring fat comprising a triacylglyceride composition as defined in any of the previous claims.

11. Use of a triacylglyceride composition as defined in any of the previous claims as a hard stock or structuring fat.

12. Use of a triacylglyceride composition according to claim 11 in the preparation of a water-continuous or fat-continuous edible emulsion.

13. Method for the preparation of a water-continuous or fat-continuous edible emulsion such as a margarine or spread comprising the steps of combining hard stock as defined in any of the previous claims, a liquid oil and an aqueous phase.

14. Margarine or margarine fat, optionally obtainable by the method of claim 13, comprising a triglyceride composition as defined in any of the previous claims.

15. Spread, optionally obtainable by the method of claim 13, comprising a triglyceride composition as defined in any of the previous claims.
